# EUROPEAN PATENT APPLICATION

(11) **EP 3 842 032 A1**
(43) Date of publication of application: **30.06.2021**
(21) Application number: 19461622.3
(22) Date of filing: 24.12.2019
(51) Int. Cl.: A61K 9/08, A61K 9/00, A61K 31/4725, C07D 405/06, A61K 47/02, A61P 27/04

(54) **A PHARMACEUTICAL FORMULATION**

(71) Applicant: Warszawskie Zaklady Farmaceutyczne Polfa S.A., 01-207 Warszawa (PL)
(72) Inventor: PIETRZAK, Tomasz, 95-020 Wisniowa Gora (PL); RATAJCZAK, Tomasz, 01-104 Warszawa (PL); KUBISIAK, Marcin, 03-983 Warszawa (PL); FALEK, Krzysztof, 03-481 Warszawa (PL); MASLOWSKA, Malgorzata, 83-000 Pruszcz Gdanski (PL); LUNIO, Rafal, 80-180 Gdansk (PL); MALIK, Katarzyna, 02-496 Warszawa (PL)
(74) Representative: Rechnio, Justyna Tatiana

(57) **Abstract**

This invention relates to an ophthalmic formulation comprising lifitegrast, an amino compound, a tonicity-adjusting agent and water, wherein the formulation has an osmolality of 150-330 mOsm/kg.

The invention also relates to lifitegrast salts comprising lifitegrast and an amino compound defined herein.

## Description

The present invention relates to a pharmaceutical formulation, particularly an ophthalmic formulation containing lifitegrast, an amino compound, a tonicity-adjusting agent and water. The present invention also relates to lifitegrast salts.

Lifitegrast is named (S)-2-(2-(benzofuran-6-carbonyl)-5,7-dichloro-1,2,3,4-tetrahydroisoquinoline-6-carboxamido)-3-(3-(methylsulfonyl)phenyl)propanoic acid and has the following structure:

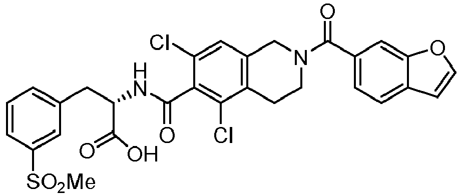

Lifitegrast binds to the integrin lymphocyte function-associated antigen-1 (LFA-1), a cell surface protein found on leukocytes, and blocks the interaction of LFA-1 with its cognate ligand (intercellular adhesion molecule-1 (ICAM-1)). ICAM-1 may be overexpressed in corneal and conjunctival tissues because of inflammation in dry eye syndrome. The LFA-1/ICAM-1 interaction can contribute to the formation of an immunological synapse resulting in T-cell activation and migration to target tissues. The use of lifitegrast is thought to disrupt this inflammatory mechanism and down-regulate inflammation mediated by T lymphocytes, which in turn stabilises the tear film.

Lifitegrast is marketed in the USA as Xiidra®. Xiidra® contains lifitegrast and is indicated for the treatment of the signs and symptoms of dry eye disease.

Xiidra® is prescribed as a 5% ophthalmic solution. The formulation contains the following components, lifitegrast (50 mg/mL), sodium chloride, sodium phosphate dibasic anhydrous, sodium thiosulfate pentahydrate, sodium hydroxide and/or hydrochloric acid (to adjust pH) and water for injection.

The ophthalmic solution is supplied as a sterile, isotonic solution of lifitegrast with an osmolality of 200-330 mOsm/kg and a pH of 7.0-8.0.

WO 2019/171260 discloses what the applicant considered to be a stable pharmaceutical composition containing lifitegrast and one or more pharmaceutical excipients including a sodium phosphate dibasic buffering agent. The composition is free of antioxidants, e.g. sodium thiosulfate and free of preservatives, e.g. benzalkonium chloride. The absence of such ingredients is said to provide a cheaper, simplified formulation without compromising the chemical stability of the product. However, the stability of the antioxidant-free formulation is not supported with a stability data.

WO 2014/100135 discloses a stable pharmaceutical composition comprising lifitegrast. However the formulations described therein require the presence of an antioxidant, preferably sodium thiosulfate and the presence of a sodium phosphate dibasic buffering agent.

The requirement for buffering agents in aqueous formulations, in particular ophthalmic formulations is well established in the pharmaceutical field. Water-soluble buffering agents serve to maintain a tolerable pH value, which provides that the formulation will not damage the area to which is administered, for example an ophthalmic solution, to a patients eye. Buffering agents also protect active ingredients from chemical degradation, e.g. through acid or base mediated hydrolysis reactions.

Unfortunately, although the use of sodium phosphate buffers remains prevalent, they can be problematic when used in ophthalmic formulations.

Recent studies have suggested that patients with a damaged cornea can develop permanent corneal opacity through scarring if they frequently use eye drops containing phosphates. It is thought that owing to the natural presence of calcium within the cornea, a chemical reaction between calcium and sodium phosphate can occur that leads to the production of insoluble calcium phosphate crystals. This calcification may lead to impaired vision because calcium salts cannot be removed by the normal fluids present within the environment of the eye. In the extreme circumstances, a patient's vision may have to be restored by transplantation of the cornea.

Consequently, there is a need to develop ophthalmic formulations with good chemical stability that are suitable for all patients, including those who may already have a damaged cornea.

Accordingly, the present invention provides an ophthalmic formulation comprising, lifitegrast; an amino compound selected from arginine, asparagine, carnosine, histidine, cysteine, lysine, methionine, tromethamine, meglumine, tryptamine, tryptophan, ornithine, N-(2-acetamido)-2-aminoethanesulfonic acid, N-(2-(acetamido)imino)diacetic acid, 2-amino-2-methyl-1-propanol, 2-amino-2-methyl-1,3-propanediol, N-(1,1-dimethyl-2-hydroxyethyl)-3-amino-2-hydroxypropanesulfonic acid, N,N-bis(2-hydroxyethyl)-2-aminoethanesulfonic acid, N,N-bis(2-hydroxyethyl)glycine, 2,2'-(propane-1,3-diyldiimino)bis[2-(hydroxymethyl)propane-1,3-diol], 2-[bis(2-hydroxyethyl)imino]-2-(hydroxymethyl)-1,3-propanediol, 2-aminoethanol and/or (2R,3R,4R,5S)-6-methylaminohexane-1,2,3,4,5-pentol, 2,2',2"-nitrilotriethanol; a tonicity-adjusting agent; and water, wherein the formulation has an osmolality of 150-330 mOsm/kg.

Advantageously, it has been found that an ophthalmic formulation containing the aforementioned components provides an environment in which the chemical stability and solubility of the active ingredient is improved, and in which the pH of the formulation is well regulated.

The present invention will now be described with reference to the accompanying drawings, in which:
- Fig. 1: shows the XRPD spectrum for lifitegrast carnosine salt;
- Fig. 2: shows the XRPD spectrum for lifitegrast histidine salt;
- Fig. 3: shows the XRPD spectrum for lifitegrast arginine salt;
- Fig. 4: shows the XRPD spectrum for lifitegrast tromethamine salt; and
- Fig. 5: shows the XRPD spectrum for lifitegrast lysine salt.

The inclusion of an amino compound selected from the list described hereinabove is particularly advantageous, because one or more of these amino compounds when present in the formulation of the invention provides pH regulation, whilst concomitantly obviating the need for a sodium phosphate buffer.

It has also been found that the inclusion of particular amino compounds selected from the list described hereinabove is even more advantageous, because one or more of these amino compounds when present in the formulation of the invention provides pH regulation, whilst concomitantly obviating the need for a sodium phosphate buffer and the need to include an antioxidant compound, for example sodium thiosulfate.

Consequently, the ophthalmic formulation of the present invention is suitable for all patients suffering from impaired vision, eye discomfort and dry eye syndrome or symptoms thereof, including those who may already have damaged cornea.

It has also been found that particular lifitegrast salts (ionic pairs) have a high solubility in water in water compared to pharmaceutically acceptable salts e.g. the sodium salt that are routinely used. This property is also particularly advantageous, because when administered to the eye, for example as a component of an ophthalmic formulation, the high water solubility of the salts supports increased permeation of lifitegrast across the membrane of the cornea. Increased permeation results in an increased local concentration of the active ingredient at the site of action. Consequently, a smaller amount of lifitegrast salt may be used to provide an equivalent therapeutic effect (compared to lifitegrast, or more routinely used pharmaceutically acceptable salts of lifitegrast).

Accordingly, the present invention also provides lifitegrast salts comprising lifitegrast and an amino compound listed hereinabove.

The present invention provides an ophthalmic formulation comprising an amino compound selected from arginine, asparagine, carnosine, histidine, cysteine, lysine, methionine, tromethamine, meglumine, tryptamine, tryptophan, ornithine, N-(2-acetamido)-2-aminoethanesulfonic acid, N-(2-(acetamido)imino)diacetic acid, 2-amino-2-methyl-1-propanol, 2-amino-2-methyl-1,3-propanediol, N-(1,1-dimethyl-2-hydroxyethyl)-3-amino-2-hydroxypropanesulfonic acid, N,N-bis(2-hydroxyethyl)-2-aminoethanesulfonic acid, N,N-bis(2-hydroxyethyl)glycine, 2,2'-(propane-1,3-diyldiimino)bis[2-(hydroxymethyl)propane-1,3-diol], 2-[bis(2-hydroxyethyl)imino]-2-(hydroxymethyl)-1,3-propanediol, 2-aminoethanol and/or (2R,3R,4R,5S)-6-methylaminohexane-1,2,3,4,5-pentol, 2,2',2"-nitrilotriethanol. The amino compounds are compounds that are able to act as a base. They all contain nitrogen atoms that can be readily protonated. Advantageously, the amino compounds are able to regulate the pH of the formulation by acting as a buffer, and obviate the need for using a sodium phosphate buffer. The amino compounds also increase the solubility of lifitegrast, and provide a formulation with good chemical stability with respect to the active ingredient.

The ophthalmic formulation also comprises a tonicity-adjusting agent. A tonicity-adjusting agent is able alter the tonicity of a solution, which is typically defined in terms of its osmolality (mOsm/kg).

The tonicity-adjusting agent may provide an isotonic, hypertonic or hypotonic solution. The tonicity properties of a solution will depend upon the identity and the amount of tonicity-adjusting agent used and the volume of the solution. The skilled person is well aware of how to adjust tonicity to a target value, for example to achieve an osmolality of 150-330 mOsm/kg.

In particular, sodium chloride may be used as the tonicity-adjusting agent. Sodium chloride may be included to provide a formulation with a tonicity or osmolality similar to, or lower than the environment of the human eye. Advantageously, balancing the tonicity of the formulation with that of the eye prevents irritation upon administration of the formulation to the eye.

The tonicity-adjusting agent may be selected from sodium chloride, sodium sulfate, polyhydroxylated alcohols for example, glycerol, mannitol or sorbitol and combinations thereof.

In one embodiment, the formulation comprises an amino compound selected from arginine, asparagine, cysteine, lysine, methionine, tromethamine, meglumine, tryptamine, tryptophan and/or ornithine.

Preferably, formulations comprising an amino compound selected from arginine, asparagine, cysteine, lysine, methionine, tromethamine, meglumine, tryptamine, tryptophan and/or ornithine may further comprise one or more pharmaceutically acceptable excipients and/or a pH modifier.

Suitable pharmaceutically acceptable excipients include, but are not limited to, ethylenediamine tetraacetic acid (EDTA), EDTA disodium, calcium disodium edetate, EDTA trisodium, EDTA tetrasodium, citric acid, sodium citrate and/or combinations thereof.

The formulations may comprise sodium EDTA. The presence of sodium EDTA advantageously prevents the formation of impurities, which would otherwise compromise the chemical stability of the formulation. Moreover, the use of sodium EDTA obviates the need for sodium thiosulfate, which is known to be an irritant to mucous membranes, and consequently is not an ideal excipient for use within ophthalmic formulations.

Alternatively, the formulations may comprise lifitegrast; an amino compound selected from arginine, asparagine, cysteine, lysine, methionine, tromethamine, meglumine, tryptamine, tryptophan and/or ornithine; a pH modifier, for example NaOH; and water, wherein the formulation has an osmolality of 150-330 mOsm/kg, and wherein the oxygen content of the water is <1% (compared to a saturated oxygen solution under the same conditions).

Deoxygenated water may be prepared by bubbling nitrogen into water. The use of deoxygenated water also obviates the need for sodium thiosulfate.

Suitable pH modifiers are inorganic bases, for example NaOH.

pH modifiers may be added to the formulation in order to provide a pH that is suitable for an ophthalmic formulation. However, depending on the amino compound (and amount of amino compound) that is used in the ophthalmic formulations of the invention, a pH modifier may not be required.

All of the formulations of the present invention described herein preferably have a pH of 7.0-8.0. Preferably, the pH of the formulations are 7.2-7.8. The pH may be controlled by the amount of amino compound included in the formulation, or by the amount of amino compound and pH modifier included within the formulation.

In one embodiment, the formulation comprises an amino compound selected from arginine, asparagine, tryptamine, lysine, tromethamine, meglumine or ornithine and a pharmaceutically acceptable excipient. In a preferred embodiment, the pharmaceutically acceptable excipient is EDTA, for example sodium EDTA.

In one embodiment, the formulation comprises an amino compound selected from cysteine, tryptophan and methionine and a pH modifier. In a preferred embodiment, the pH modifier is NaOH.

In a further embodiment, the formulation comprises an amino compound selected from carnosine or histidine.

Advantageously, carnosine and histidine are capable of acting as a buffer, increasing the solubility of lifitegrast and providing an antioxidant effect to the formulation. The triple action effect of these amino compounds obviates the need for using a sodium phosphate buffer, and reduces the need to include additional pharmaceutically acceptable excipients, for example EDTA, and the need to include pH modifiers.

All of the formulations of the present invention described herein comprise lifitegrast and an amino compound.

In one embodiment, the molar ratio of the amino compound to lifitegrast is from 0.05:1 to 3:1, in a preferred embodiment, the molar ratio of the arginine, asparagine, cysteine, lysine, methionine, tromethamine, meglumine, tryptamine, tryptophan, ornithine, carnosine or histidine to lifitegrast is from 0.05:1 to 3:1.

For example, the molar ratio of arginine to lifitegrast may be from 0.05:1 to 3:1, or the molar ratio of asparagine to lifitegrast may be from 0.05:1 to 3:1, or the molar ratio of cysteine to lifitegrast may be from 0.05:1 to 3:1, or the molar ratio of lysine to lifitegrast may be from 0.05:1 to 3:1, or the molar ratio of methionine to lifitegrast may be from 0.05:1 to 3:1, or the molar ratio of tromethamine to lifitegrast may be from 0.05:1 to 3:1, or the molar ratio of meglumine to lifitegrast may be from 0.05:1 to 3:1, or the molar ratio of tryptamine to lifitegrast may be from 0.05:1 to 3:1, or the molar ratio of tryptophan to lifitegrast may be from 0.05:1 to 3:1, or the molar ratio of ornithine to lifitegrast may be from 0.05:1 to 3:1, or the molar ratio of carnosine to lifitegrast may be from 0.05:1 to 3:1 or the molar ratio of histidine to lifitegrast may be from 0.05:1 to 3:1.

In a further embodiment, the molar ratio of the amino compound to lifitegrast is from 0.05:1 to 2:1, in a preferred embodiment, the molar ratio of the arginine, asparagine, cysteine, lysine, methionine, tromethamine, meglumine, tryptamine, tryptophan, ornithine, carnosine or histidine to lifitegrast is from 0.05:1 to 2:1.

For example, the molar ratio of arginine to lifitegrast may be from 1:1 to 2:1, or the molar ratio of asparagine to lifitegrast may be from 1:1 to 2:1, or the molar ratio of cysteine to lifitegrast may be from 1:1 to 2:1, or the molar ratio of lysine to lifitegrast may be from 1:1 to 2:1, or the molar ratio of methionine to lifitegrast may be from 1:1 to 2:1, or the molar ratio of tromethamine to lifitegrast may be from 1:1 to 2:1, or the molar ratio of meglumine to lifitegrast may be from 0.05:1 to 3:1, or the molar ratio of tryptamine to lifitegrast may be from 0.05:1 to 3:1, or the molar ratio of tryptophan to lifitegrast may be from 0.05:1 to 3:1, or the molar ratio of ornithine to lifitegrast may be from 0.05:1 to 3:1, or the molar ratio of carnosine to lifitegrast may be from 1:1 to 2:1 or the molar ratio of histidine to lifitegrast may be from 1:1 to 2:1.

In one embodiment, the amino compound is preferably present in a molar amount that exceeds the molar amount of lifitegrast present in the formulation. Advantageously the use of a super stoichiometric amount of the amino compound provides advantageous solubility effects with respect to lifitegrast, and advantageous effects that increases the chemical stability of the lifitegrast.

In one embodiment, the amino compound is preferably present in a molar amount that does exceed the molar amount of lifitegrast present in the formulation. In such embodiments the amino compound is used a sub stoichiometric amount to provide an antioxidant effect to the formulation. In such embodiments, a pH-modifier, for example NaOH may be added to provide advantageous solubility effects.

Alternatively, an additional amino compound can be added to adjust the pH of the formulation as required.

In one embodiment, the ophthalmic formulation comprises lifitegrast, an amino compound selected from carnosine or histidine, a tonicity-adjusting agent and water, wherein the formulation has an osmolality of 150-330 mOsm/kg.

In one embodiment, the ophthalmic formulation comprises lifitegrast, an amino compound selected from arginine, asparagine, cysteine, lysine, methionine, tromethamine, meglumine, tryptamine, tryptophan or ornithine a tonicity-adjusting agent and water, wherein the formulation has an osmolality of 150-330 mOsm/kg and wherein the formulation optionally further comprises EDTA and/or NaOH.

In a preferred embodiment, the ophthalmic formulations of the present invention have an osmolality of 150-330 mOsm/kg.

In a preferred embodiment, the ophthalmic formulation may comprise lifitegrast, arginine, sodium EDTA, a tonicity-adjusting agent and water, wherein the formulation has an osmolality of 150-330 mOsm/kg.

In a preferred embodiment, the ophthalmic formulation may comprise lifitegrast, asparagine, sodium EDTA, a tonicity-adjusting agent and water, wherein the formulation has an osmolality of 150-330 mOsm/kg.

In a preferred embodiment, the ophthalmic formulation may comprise lifitegrast, cysteine, NaOH, a tonicity-adjusting agent and water, wherein the formulation has an osmolality of 150-330 mOsm/kg.

In a preferred embodiment, the ophthalmic formulation may comprise lifitegrast, lysine, sodium EDTA, a tonicity-adjusting agent and water, wherein the formulation has an osmolality of 150-330 mOsm/kg.

In a preferred embodiment, the ophthalmic formulation may comprise lifitegrast, methionine, NaOH, a tonicity-adjusting agent and water, wherein the formulation has an osmolality of 150-330 mOsm/kg.

In a preferred embodiment, the ophthalmic formulation may comprise lifitegrast, tromethamine, sodium EDTA, a tonicity-adjusting agent and water, wherein the formulation has an osmolality of 150-330 mOsm/kg.

In a preferred embodiment, the ophthalmic formulation may comprise lifitegrast, meglumine, sodium EDTA, a tonicity-adjusting agent and water, wherein the formulation has an osmolality of 150-330 mOsm/kg.

In a preferred embodiment, the ophthalmic formulation may comprise lifitegrast, tryptamine, sodium EDTA, a tonicity-adjusting agent and water, wherein the formulation has an osmolality of 150-330 mOsm/kg.

In a preferred embodiment, the ophthalmic formulation may comprise lifitegrast, tryptophan, NaOH, a tonicity-adjusting agent, water, and optionally EDTA, wherein the formulation has an osmolality of 150-330 mOsm/kg.

In a preferred embodiment, the ophthalmic formulation may comprise lifitegrast, ornithine, sodium EDTA, a tonicity-adjusting agent and water, wherein the formulation has an osmolality of 150-330 mOsm/kg.

In a preferred embodiment, the ophthalmic formulation may comprise lifitegrast, carnosine, a tonicity-adjusting agent and water, wherein the formulation has an osmolality of 150-330 mOsm/kg.

In a preferred embodiment, the ophthalmic formulation may comprise lifitegrast, histidine, a tonicity-adjusting agent and water, wherein the formulation has an osmolality of 150-330 mOsm/kg.

In the aforementioned embodiments, the tonicity-adjusting agent is preferably sodium chloride.

The ophthalmic formulation may comprise lifitegrast, carnosine or histidine, and a further amino compound selected from cysteine, methionine and tryptophan, a tonicity-adjusting agent and water, wherein the formulation has an osmolality of 150-330 mOsm/kg.

The ophthalmic formulation may comprise lifitegrast, cysteine, and a further amino compound selected from tromethamine, meglumine, arginine, asparagine, lysine and ornithine, a tonicity-adjusting agent and water, wherein the formulation has an osmolality of 150-330 mOsm/kg. This formulation preferably also contains NaOH.

The ophthalmic formulation may comprise lifitegrast, methionine, and a further amino compound selected from tromethamine, meglumine, arginine, asparagine, lysine and ornithine, a tonicity-adjusting agent and water, wherein the formulation has an osmolality of 150-330 mOsm/kg. This formulation preferably also contains NaOH.

The ophthalmic formulation may comprise lifitegrast, tryptophan, and a further amino compound selected from tromethamine, meglumine, arginine, asparagine, lysine and ornithine, a tonicity-adjusting agent and water, wherein the formulation has an osmolality of 150-330 mOsm/kg. This formulation preferably also contains NaOH.

Advantageously, the use of two amino compounds within the ophthalmic formulations of the invention provides oxygen-resistant formulations that are even more stable compared to some ophthalmic formulations containing one amino compound.

Optionally, the formulations of the present invention noted as preferred embodiments hereinabove may comprise one or more pharmaceutically acceptable excipients and/or a pH modifier (if not already included). If a pH modifier is to be included within the formulation, NaOH is preferably used, however other pharmaceutically acceptable inorganic bases may be suitable.

The formulation may further comprise one or more additional pharmaceutically acceptable excipients typically used within ophthalmic formulations.

The formulation of the present invention is preferably packaged in a multi-dose receptacle, for example a bottle, an ophthalmic squeeze dispenser or an ophthalmic pump dispenser, or a single-dose single use receptacle, for example an ampoule or snap-off plastic vial.

The ophthalmic formulations of the present invention are for use in the treatment of dry eye syndrome which is also known as dry eye disease or keratoconjunctivitis sicca. Typically, the formulation is for administration to the eye(s) of a human patient, if the tear film is unbalanced due to excessive evaporation or insufficient tear production which causes the eye(s) to be itchy, sore, gritty, red, blurry, sensitive to light or excessively watery.

In one embodiment, the ophthalmic formulations of the present invention are for use in the treatment of dry eye syndrome in a patient with a damaged cornea.

The present invention also provides salts of lifitegrast, comprising lifitegrast and an amino compound as defined herein.

In a preferred embodiment, the present invention provides lifitegrast arginine salt, lifitegrast asparagine salt, lifitegrast cysteine salt, lifitegrast lysine salt, lifitegrast methionine salt, lifitegrast tromethamine salt, lifitegrast meglumine salt, lifitegrast tryptamine salt, lifitegrast tryptophan salt, lifitegrast ornithine salt, lifitegrast carnosine salt and lifitegrast histidine salt.

By way of an example, lifitegrast asparagine salt has the following formula:

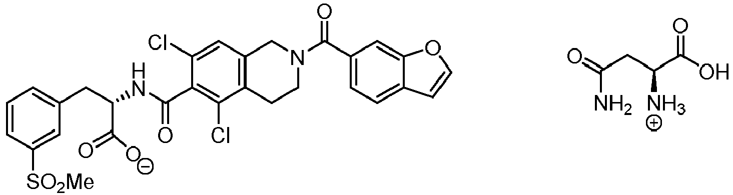

Other salts have analogous formulae.

Advantageously, these salts have enhanced water solubility compared to lifitegrast, and provide useful buffering and antioxidant effects when dissolved in water. Additionally, their use in ophthalmic formulations obviates the need for the use of additional excipients, for example chelating agents, antioxidants and pH modifiers.

Synthetic protocols to prepare lifitegrast are known in the art, see for example WO 2019/053607.

The formulations of the present invention may be prepared using the established protocols of the art.

Typically, lifitegrast, an amino compound and a tonicity-adjusting agent are added to deionised water and mixed until the respective components dissolve. The deionised water may also be deoxygenated (oxygen concentration <1%) as described hereinabove.

Alternatively, a lifitegrast salt comprising lifitegrast and an amino compound selected from arginine, asparagine, carnosine, histidine, cysteine, lysine, methionine, tromethamine, meglumine, tryptamine, tryptophan, ornithine, N-(2-acetamido)-2-aminoethanesulfonic acid, N-(2-(acetamido)imino)diacetic acid, 2-amino-2-methyl-1-propanol, 2-amino-2-methyl-1,3-propanediol, N-(1,1-dimethyl-2-hydroxyethyl)-3-amino-2-hydroxypropanesulfonic acid, N,N-bis(2-hydroxyethyl)-2-aminoethanesulfonic acid, N,N-bis(2-hydroxyethyl)glycine, 2,2'-(propane-1,3-diyldiimino)bis[2-(hydroxymethyl)propane-1,3-diol], 2-[bis(2-hydroxyethyl)imino]-2-(hydroxymethyl)-1,3-propanediol, 2-aminoethanol or (2R,3R,4R,5S)-6-methylaminohexane-1,2,3,4,5-pentol, 2,2',2"-nitrilotriethanol may be prepared by crystallisation or precipitation to provide a lifitegrast salt in crystalline or amorphous form.

The crystalline or amorphous lifitegrast/amino compound salt can then be used directly to prepare an ophthalmic formulation.

The use of crystalline or amorphous lifitegrast/amino compound salts to prepare an ophthalmic formulation is particularly advantageous compared to preparing an ophthalmic formulation by adding lifitegrast and an amino compound separately to water. This is because preparation of the crystalline or amorphous lifitegrast/amino compound salt serves to purify lifitegrast from impurities that cannot undergo salt formation with the amino compound. Consequently, the use of crystalline or amorphous lifitegrast/amino compound salts in ophthalmic formulations provides lifitegrast in a higher purity.

The lifitegrast salt may be prepared using a 1:1 molar ratio of lifitegrast and amino compound or an excess of the amino compound (not exceeding a 1:3 molar ratio of lifitegrast and amino compound). Once prepared, the lifitegrast salt may be added to deionised and optionally deoxygenated water, followed by a tonicity-adjusting agent, for example sodium chloride and optionally any pharmaceutically acceptable excipient and/or a pH modifier.

The aqueous formulation may then be further sterilised, for example by bubbling further nitrogen through the solution, and then aseptically filtering the solution.

The present invention will now be described with reference to the following examples, which are not intended to be limiting.

### Examples

### Example 1: Preparation of an ophthalmic formulation using lifitegrast

Deionised water (15 mL) and an amino compound selected from arginine, asparagine, cysteine, lysine, methionine, tromethamine, meglumine, tryptamine, tryptophan, ornithine, carnosine and histidine in an amount of 0.52 to 1.62 mmol is added to a vessel equipped with a magnetic stirrer at room temperature (20-25°C) and mixed for 10 minutes. To the mixture, lifitegrast is added in an amount of 0.54 to 1.62 mmol, and the mixture is allowed to stir. Sodium chloride is then added (0.1 to 10 mg/mL) to the mixture, and then edetate salt (0.00059 to 0.06 mmol) and the mixture is allowed to stir. At this point, the pH of the formulation is measured. The pH of the formulation is adjusted to pH 7.3 with additional amino compound. Alternatively, the pH can be adjusted to pH 7.3 with the addition of sodium hydroxide. After the pH adjustment, the aqueous solution is inspected for particulate matter and aseptically filtered (membrane pore diameter 0.22 µm).

### Example 2: Preparation of an ophthalmic formulation using amorphous lifiteqrast carnosine salt

### Preparation of lifitegrast carnosine salt

18 mL of water and 345 mg of carnosine were added to a vessel equipped with a magnetic stirrer at room temperature (20-25°C) and mixed for 10 minutes. To the mixture, 960 mg of lifitegrast was then added. The mixture was stirred for 60 minutes (until the solution was clear) and was then filtered and evaporated to dryness.

### Preparation of amorphous lifitegrast carnosine salt (method I)

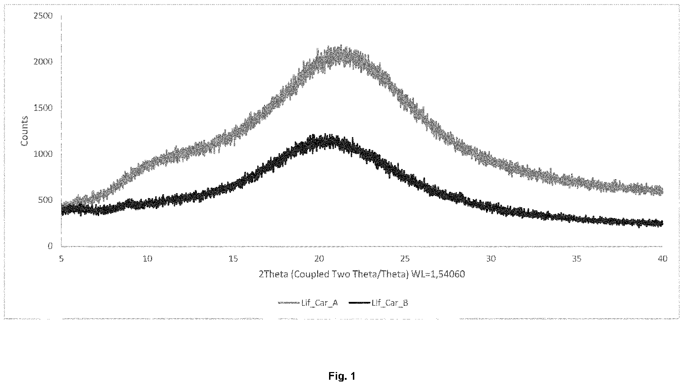

Lifitegrast carnosine salt (438 mg) was added to 96% ethanol (3 mL). The mixture was heated to 60-65°C and stirred for 60 minutes at this temperature. The solution was then cooled to 5-8°C. The resulting white precipitate was removed by filtration, washed with cold absolute ethanol, and dried to provide amorphous lifitegrast carnosine salt (85.4% yield). The PXRD of the dried carnosine salt was recorded confirming amorphous form of salt (see Figure 1, Lif_Car_A).

### Preparation of amorphous lifitegrast carnosine salt (method II)

Lifitegrast carnosine salt (438 mg) was added to a mixture of ethanol and isopropanol (3:7 v/v). The mixture was heated to 60-65°C and stirred for 60 minutes at this temperature. The solution was then cooled to 5-8°C. The resulting white precipitate was removed by filtration, washed with cold absolute ethanol, and dried to provide amorphous lifitegrast carnosine salt (95.2% yield). The PXRD of the dried carnosine salt was recorded confirming amorphous form of salt (see Figure 1, Lif_Car_B).

### Preparation of an ophthalmic formulation using amorphous lifitegrast carnosine salt

Deionised water (15 mL) and amorphous lifitegrast carnosine salt (1.62 mmol) are added to a vessel equipped with a magnetic stirrer at room temperature (20-25°C) and mixed for 10 minutes. Sodium chloride (0.1 to 10.0 mg/mL) is then added to the mixture, and the mixture is allowed to stir. At this point, the pH of the formulation is measured. If the pH of the formulation is too acidic (pH outside 7.2-7.8) then carnosine is added to adjust the pH to between 7.2-7.8. After the pH adjustment, the aqueous solution is inspected for particulate matter and filtered if deemed necessary. Alternately, the pH can be adjusted with the addition of sodium hydroxide. The aqueous solution (or filtrate) is then aseptically filtered.

Alternatively, deionised water (15 mL) and amorphous lifitegrast carnosine salt (1.62 mmol) are added to a vessel equipped with a magnetic stirrer at room temperature (20-25°C) and mixed for 10 minutes. Sodium chloride (0.1 to 10.0 mg/mL) is then added to the mixture, and the mixture is allowed to stir. At this point, the pH of the formulation is measured. If the pH of the formulation is too acidic (pH outside 7.2-7.8) then carnosine is added to adjust the pH to between 7.2-7.8. Then the solution is sparged with nitrogen until the oxygen concentration is less than 1% to a saturated oxygen solution under these conditions. The aqueous solution is then inspected for particulate matter and filtered if deemed necessary. Alternately, the pH can be adjusted with the addition of sodium hydroxide. The aqueous solution (or filtrate) is then aseptically filtered.

### Example 3: Preparation of an ophthalmic formulation using amorphous lifitegrast arginine salt

### Preparation of lifitegrast arginine salt

6.0 mL of water and 86 mg of arginine were added to a vessel equipped with a magnetic stirrer at room temperature (20-25°C) and mixed for 10 minutes. To the mixture, 303 mg of lifitegrast was then added. The mixture was stirred for 60 minutes (until the solution was clear) and was then filtered and evaporated to dryness.

### Preparation of amorphous lifitegrast arginine salt

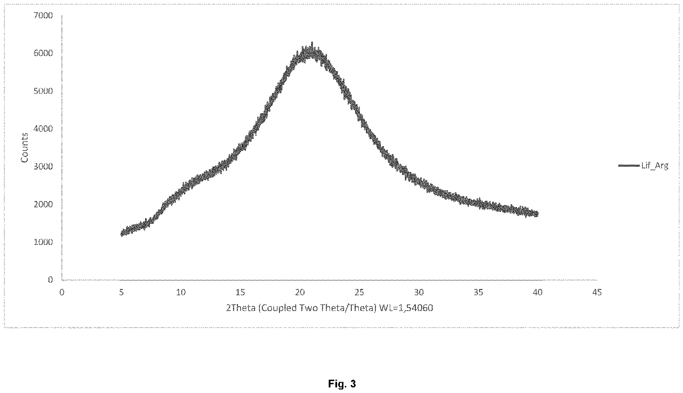

Lifitegrast arginine salt was added to a mixture of 96% ethanol (3 mL) and isopropanol (3:0.3 v/v). The mixture was heated to 60-65°C and stirred for 15 minutes at this temperature. The reaction mass was then cooled to 5-8°C. The solid precipitate was removed by filtration and washed with isopropanol. The wet material was moved to a vial and dried to provide amorphous lifitegrast arginine salt. The PXRD of the dried arginine salt was recorded confirming amorphous form of salt (Figure 3, Lif_Arg) to provide amorphous lifitegrast arginine salt.

### Preparation of an ophthalmic formulation using amorphous lifitegrast arginine salt

Deionised water (15 mL) and amorphous lifitegrast arginine salt (1.62 mmol) are added to a vessel equipped with a magnetic stirrer at room temperature (20-25°C) and mixed for 10 minutes. Sodium chloride is then added to the mixture, and edetate salt (0.00059 to 0.06 mmol) and the mixture is allowed to stir. At this point, the pH of the formulation is measured. If the pH of the formulation is too acidic (pH outside 7.2-7.8) then arginine is added to adjust the pH to between 7.2-7.8. Alternatively, the pH can be adjusted by addition of sodium hydroxide. After the pH adjustment, the aqueous solution is inspected for particulate matter and filtered if deemed necessary. The aqueous solution (or filtrate) is then aseptically filtered.

### Example 4: Preparation of amorphous lifitegrast histidine salt

### Preparation of lifitegrast histidine salt

3.0 mL of water and 59 mg of histidine were added to a vessel equipped with a magnetic stirrer at room temperature (20-25°C) and mixed for 10 minutes. To the mixture, 151 mg of lifitegrast was then added. The mixture was stirred for 60 minutes (until the solution was clear) and was then filtered and evaporated to dryness.

### Preparation of amorphous lifitegrast histidine salt

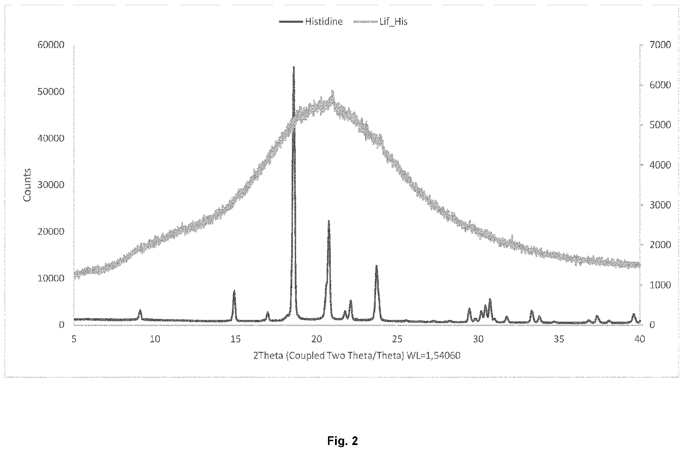

Lifitegrast histidine salt was added to a mixture of water (50 µL) and isopropanol (1450 µL). The mixture was heated to 72-75°C and stirred for 15 minutes at this temperature. Excess histidine (white precipitate) was removed by filtration. The reaction mass was allowed to cool at the room temperature (20-25 °C) under stirring. The solid precipitate was removed by filtration and washed with isopropanol. The wet material was moved to a vial and dried to provide amorphous lifitegrast histidine salt. The PXRD of the dried histidine salt was recorded confirming amorphous form of salt (Figure 2, Lif_His).

### Example 5: Preparation of amorphous and crystalline lifitegrast tromethamine salts

### Preparation of lifitegrast tromethamine salt

3.0 mL of water and 60 mg of tromethamine were added to a vessel equipped with a magnetic stirrer at room temperature (20-25°C) and mixed for 10 minutes. To the mixture, 305 mg of lifitegrast was then added. The mixture was stirred for 60 minutes (until the solution was clear) and was then filtered and evaporated to dryness.

### Preparation of amorphous lifitegrast tromethamine salt

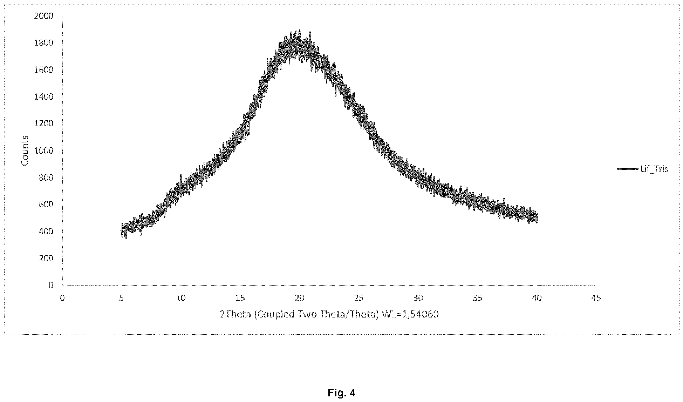

Lifitegrast tromethamine salt was added to a mixture of water and isopropanol (1:9 v/v). The mixture was heated to 60-65°C and stirred for 15 minutes at this temperature. The reaction mass was allowed to cool at the room temperature (20-25°C) and stirred for 30 minutes. The solid was removed by filtration and washed with isopropanol, and dried. The PXRD of the dried tromethamine salt was recorded confirming amorphous form of salt (Figure 4, Lif_Tris).

### Preparation of crystalline lifitegrast tromethamine salt

Lifitegrast tromethamine salt was added to a mixture of methanol and isopropanol (12 mL:1 mL). The mixture was heated to 60-65°C and stirred for 30 minutes at this temperature. The solution was then allowed to cool to room temperature (20-25°C) overnight. The resulting white precipitate was filtered, washed with isopropanol and dried.

### Preparation of an ophthalmic formulation using amorphous or crystalline lifitegrast tromethamine salt

Deionised water (15 mL) and amorphous or crystalline lifitegrast tromethamine salt (1.62 mmol) are added to a vessel equipped with a magnetic stirrer at room temperature (20-25°C) and mixed for 10 minutes. Sodium chloride (0.1 to 10.0 mg/mL) is then added to the mixture, and the mixture is allowed to stir. At this point, the pH of the formulation is measured. If the pH of the formulation is too acidic (pH outside 7.2-7.8) then tromethamine is added to adjust the pH to between 7.2-7.8. Alternately, the pH can be adjusted with the addition of sodium hydroxide. After the pH adjustment, the aqueous solution is inspected for particulate matter and filtered if deemed necessary. Alternately, pH can be adjusted with the addition of sodium hydroxide. The aqueous solution (or filtrate) is then aseptically filtered.

Alternatively, deionised water (15 mL) and lifitegrast tromethamine salt (1.62 mmol) are added to a vessel equipped with a magnetic stirrer at room temperature (20-25°C) and mixed for 10 minutes. Sodium chloride (0.1 to 10.0 mg/mL) and then edetate salt (0.00059 to 0.06 mmol) are added to the mixture, and the mixture is allowed to stir. At this point, the pH of the formulation is measured. If the pH of the formulation is too acidic (pH outside 7.2-7.8) then tromethamine is added to adjust the pH to between 7.2-7.8. After the pH adjustment, the aqueous solution is inspected for particulate matter and filtered if deemed necessary. Alternately, the pH can be adjusted with the addition of sodium hydroxide. The aqueous solution (or filtrate) is then aseptically filtered.

### Example 6: Preparation of amorphous lifitegrast lysine salt

### Preparation of lifitegrast lysine salt

3.5 mL of water and 72 mg of lysine were added to a vessel equipped with a magnetic stirrer at room temperature (20-25°C) and mixed for 10 minutes. To the mixture, 305 mg of lifitegrast was then added.

The mixture was stirred for 60 minutes (until the solution was clear) and was then filtered and evaporated to dryness.

### Preparation of amorphous lifitegrast lysine salt

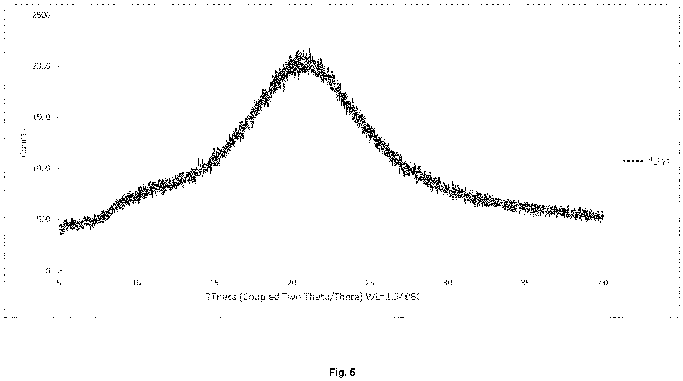

Lifitegrast lysine salt was added to a mixture of water and isopropanol (1:9 v/v). The mixture was heated to 60-65°C and stirred for 15 minutes at this temperature. The reaction mass was allowed to cool at the room temperature (20-25°C) and stirred for 30 minutes. The solid was removed by filtration and washed with isopropanol, and dried. The PXRD of the dried tromethamine salt was recorded confirming amorphous form of salt (Figure 5, Lif_Lys).

### Preparation of an ophthalmic formulation using amorphous lifitegrast lysine salt

Deionised water (15 mL) and amorphous lifitegrast lysine salt (1.62 mmol) are added to a vessel equipped with a magnetic stirrer at room temperature (20-25°C) and mixed for 10 minutes. Sodium chloride (0.1 to 10.0 mg/mL) is then added to the mixture, and the mixture is allowed to stir. At this point, the pH of the formulation is measured. If the pH of the formulation is too acidic (pH outside 7.2-7.8) then lysine is added to adjust the pH to between 7.2-7.8. Alternately, the pH can be adjusted with the addition of sodium hydroxide. After the pH adjustment, the aqueous solution is inspected for particulate matter and filtered if deemed necessary. Alternately, pH can be adjusted with the addition of sodium hydroxide. The aqueous solution (or filtrate) is then aseptically filtered.

Alternatively, deionised water (15 mL) and lifitegrast lysine salt (1.62 mmol) are added to a vessel equipped with a magnetic stirrer at room temperature (20-25°C) and mixed for 10 minutes. Sodium chloride (0.1 to 10.0 mg/mL) and then edetate salt (0.00059 to 0.06 mmol) are added to the mixture, and the mixture is allowed to stir. At this point, the pH of the formulation is measured. If the pH of the formulation is too acidic (pH outside 7.2-7.8) then lysine is added to adjust the pH to between 7.2-7.8. After the pH adjustment, the aqueous solution is inspected for particulate matter and filtered if deemed necessary. Alternately, the pH can be adjusted with the addition of sodium hydroxide. The aqueous solution (or filtrate) is then aseptically filtered.

All samples were tested in a range 5° to 40° 2θ by X-ray powder diffractometer D2 Phaser (Bruker). Samples were analysed using zero diffraction holder.

## Claims

1. An ophthalmic formulation comprising, lifitegrast; an amino compound selected from arginine, asparagine, carnosine, histidine, cysteine, lysine, methionine, tromethamine, meglumine, tryptamine, tryptophan, ornithine N-(2-acetamido)-2-aminoethanesulfonic acid, N-(2-(acetamido)imino)diacetic acid, 2-amino-2-methyl-1-propanol, 2-amino-2-methyl-1,3-propanediol, N-(1,1-dimethyl-2-hydroxyethyl)-3-amino-2-hydroxypropanesulfonic acid, N,N-bis(2-hydroxyethyl)-2-aminoethanesulfonic acid, N,N-bis(2-hydroxyethyl)glycine, 2,2'-(propane-1,3-diyldiimino)bis[2-(hydroxymethyl)propane-1,3-diol], 2-[bis(2-hydroxyethyl)imino]-2-(hydroxymethyl)-1,3-propanediol, 2-aminoethanol and/or (2R,3R,4R,5S)-6-methylaminohexane-1,2,3,4,5-pentol, 2,2',2"-nitrilotriethanol; a tonicity-adjusting agent; and water, wherein the formulation has an osmolality of 150-330 mOsm/kg.

2. The ophthalmic formulation as claimed in claim 1, wherein the amino compound is arginine, asparagine, cysteine, lysine, methionine, tromethamine, meglumine, tryptamine, tryptophan and/or ornithine.

3. The ophthalmic formulation as claimed in claim 1 or 2 further comprising a pharmaceutically acceptable excipient and/or a pH modifier, for example NaOH.

4. The ophthalmic formulation as claimed in claim 3, wherein the pharmaceutically acceptable excipient is ethylenediamine tetraacetic acid (EDTA), for example sodium EDTA.

5. The ophthalmic formulation as claimed in claim 1, wherein the amino compound is carnosine or histidine.

6. The ophthalmic formulation as claimed in any preceding claim, wherein the molar ratio of the amino compound to lifitegrast is from 0.05:1 to 3:1.

7. The ophthalmic formulation as claimed in claim 6, wherein the molar ratio of the amino compound to lifitegrast is from 0.05:1 to 2:1.

8. An ophthalmic formulation as claimed in claim 1 or 5 comprising lifitegrast, an amino compound selected from carnosine or histidine, a tonicity-adjusting agent and water, wherein the formulation has an osmolality of 150-330 mOsm/kg.

9. An ophthalmic formulation as claimed in claim 1 or 2 comprising lifitegrast, an amino compound selected from arginine, asparagine, cysteine, lysine, methionine or tromethamine, a tonicity-adjusting agent and water, wherein the formulation has an osmolality of 150-330 mOsm/kg and wherein the formulation optionally further comprises EDTA and/or NaOH.

10. An ophthalmic formulation as claimed in claim 1, 8 or 9 wherein the osmolality of the formulation is 150-330 mOsm/kg.

11. An ophthalmic formulation as claimed in any preceding claim wherein the pH of the formulation is from 7.2-7.8.

12. A multi-dose and/or single-dose receptacle comprising the ophthalmic formulation as claimed in any preceding claim.

13. The ophthalmic formulation as claimed in any preceding claim for use in the treatment of dry eye syndrome.

14. The ophthalmic formulation as claimed in claim 13 for use in the treatment of dry eye syndrome in a patient with a damaged cornea.

15. A lifitegrast salt comprising lifitegrast and an amino compound as defined in claim 1.
